# EUROPEAN PATENT SPECIFICATION

(11) **EP 4 384 314 B1**
(45) Date of publication and mention of the grant of the patent: **03.09.2025**
(21) Application number: 22741256.6
(22) Date of filing: 05.07.2022
(51) Int. Cl.: B01J 23/644, B01J 23/72, B01J 23/28, B01J 23/22, B01J 35/00, B01J 37/02, B01J 37/00, C07C 51/265

(54) **MULTILAYER CATALYTIC BED FOR THE PRODUCTION OF PHTHALIC ANHYDRIDE**
MEHRSCHICHTIGES KATALYSATORBETT ZUR HERSTELLUNG VON PHTHALSÄUREANHYDRID
LIT CATALYTIQUE MULTICOUCHE POUR LA PRODUCTION D'ANHYDRIDE PHTALIQUE

(30) Priority: 11.08.2021 IT 202100021746
(43) Date of publication of application: 19.06.2024
(73) Proprietor: POLYNT S.P.A., 24020 Scanzorosciate, (IT)
(72) Inventor: CORTELLI, Carlotta, 40122 Bologna (IT); GRAZIA, Lorenzo, 24124 Bergamo (IT); FRATALOCCHI, Laura, 20131 Milano (IT)
(74) Representative: Modiano, Micaela Nadia
(86) International application number: PCT/EP2022/068639
(87) International publication number: WO 2023/016706

(56) References cited:
- DE-A1- 102013 000 647
- US-A1- 2006 276 661

## Description

The present invention relates to a multilayer catalytic bed for the oxidation of o-xylene, naphthalene, or a mixture thereof to phthalic anhydride in the gas phase. The catalytic bed is characterized by an increased yield of phthalic anhydride and by a reduced deterioration of the catalytic performance as the catalytic bed ages. The invention also relates to a process for the production of phthalic anhydride employing the above catalytic bed.

Phthalic anhydride is an important chemical product used for the production of plasticizers, alkyd resins, unsaturated polyester resins and other commercial products. The production of phthalic anhydride is estimated at 5000 ktons/year, of which approximately 85% is produced industrially by selective oxidation of o-xylene.

For the purposes of commercial production, the oxidation is carried out in multitubular fixed-bed reactors, fed with a mixture of the starting hydrocarbon (o-xylene and/or naphthalene) and of an oxygen-containing gas, usually air. The latest-generation reactors are designed to have up to a maximum number of 30000 tubes, in order to ensure a productivity of up to 110 ktons/year of phthalic anhydride. The removal of the heat generated by the exothermic oxidation reaction, as well as the control of the reaction temperatures and the stage of completion thereof, are ensured by suitable cooling media, usually molten salts, which circulate in a jacket around the tubes of the reactor.

The catalyst which is loaded into the tubes is usually supported, i.e. the catalytically active material is deposited on an inert support, for example ceramic, which has the form of rings or alternatively granules (spherical or irregular in shape), pellets or cylinders. The catalytically active material used in the production of phthalic anhydride is generally a mixture of V₂O₅ (vanadium oxide) and TiO₂ (titanium oxide or titanium dioxide), whence the name vanadium and titanium mixed oxide, or "VTiO", catalyst. This mixture further comprises various additional components, known as dopants, which act as promoters of activity and/or selectivity. Commonly-used dopants include for example elements like cesium, antimony, potassium, phosphorous or mixtures thereof.

In current configurations, the catalytic bed is no longer constituted by a single VTiO catalyst. The current generation of catalytic systems for the selective oxidation of o-xylene and/or naphthalene to phthalic anhydride in the gase phase, in fact, uses catalytic beds that have at least four successive layers of VTiO catalyst, each layer having a different height and composition of the catalytically active material. The reason for this choice lies in the complexity of the reaction mechanism of the conversion of o-xylene and naphthalene to phthalic anhydride, which involves a great number of parasitic reactions leading to both over-oxidized byproducts (maleic anhydride and COₓ), and partially-oxidized byproducts (phtalide, o-toluic aldehyde, o-toluic acid). Such byproducts are undesired, because they decrease the yield of phthalic anhydride and in some cases they are difficult to remove. It is for this reason that multilayer configurations of the catalytic bed are adopted at industrial level, using layers of different height and varying the composition of the catalytically active material with the addition of different promoters, in order to increase the catalytic activity, thus achieving a conversion of the starting hydrocarbon that is as high as possible, and maintaining a high selectivity for phthalic anhydride, thereby minimizing the formation of undesired byproducts.

At the start of their lifespan, the current generation of catalytic systems is capable of producing 114.6 kg of phthalic anhydride per 100 kg of starting hydrocarbons fed into the plant. The lifespan (or running time) of the catalysts inside the reactor is usually 48-54 months, during which the decay of the catalytic performance translates into a loss in the weight yield of phthalic anhydride by 1% every 12 months.

In light of the above, there is a need to improve the process of production of phthalic anhydride from o-xylene and/or naphthalene, increasing the yield of phthalic anhydride and at the same time minimizing the decay of the catalytic performance as the lifespan increases.

In the scientific literature, the selective oxidation reaction of o-xylene and naphthalene in the gas phase has been studied since the 1980s and 1990s. The studies have concentrated especially on explaining the structure of the active phase of the VTiO catalyst, which as mentioned consists of the mixture of V₂O₅ and TiO₂, and on the oxidation mechanism whereby o-xylene and/or naphthalene are converted to phthalic anhydride [Saleh R., Wachs I., Chan S. et al., J. Catal., 1986, 98, 102-114], but also on the role of promoters (for example Na, K, Rb, Cs, Sb, Sn, Nb, P and Si), which are added in small quantities in order to increase selectivity toward the formation of phthalic anhydride and, at the same time, limit the presence of reaction byproducts and to minimize the total combustion producing CO and CO₂ [Annibali S., Cavani F., Guerrini A., Panzacchi B., Trifirò F., Fumagalli C., Leanza R., Mazzoni G., Catal. Today, 2003, 78, 117].

In the patent literature, particular importance has also been accorded to the role of promoters to maximize catalytic performance. If the composition of the reaction flow is changed as a function of its stage of advancement, it becomes necessary in fact to adapt the chemical/physical properties of the active phase (in terms both of percentage of active phase, and of content of promoters) in order to obtain the best catalytic performance. In this regard, prior art documents US 6774246, EP 1063222 and WO 2005/011862 are cited by way of example, in which the content of V₂O₅ decreases going from the upper layers to the lower layers in order to limit exothermicity problems, the content of cesium (selectivity promoter) also decreases going to the lower layers, while antimony (activity promoter) increases in the lower layers of the catalytic bed, with the aim of achieving a conversion of the starting hydrocarbon that is as complete as possible.

In parallel with the optimization of the composition of the active phase of the different layers that make up the catalytic bed, new promoters have been studied. For example, EP 2027102 discloses a catalytic bed with three layers, the first of which consists of an inert material, the second of a bulk catalyst having its active phase consisting of Ce_{0.02}Ag_{0.71}V₂Oₓ, and the lowest layer consists of ceramic rings coated with active phase VTiO promoted with Cs, Sb and P. On the other hand, DE 102008044890 discloses a catalytic bed with four layers consisting of ceramic rings coated with active phase VTiO, for which BiVO₄, AgVO₃, Ag₂WO₄ are studied as promoters for the first layer. US 2006/276661 A1 and DE 10 2013 000647 A1 disclose a catalytic bed comprising four catalytic layers of vanadium and titanium mixed oxide (VTiO) catalyst arranged in series with respect to the flow of a gaseous feed mixture comprising the hydrocarbon and an oxygen-containing gas, so that the gaseous feed mixture flows in sequence through a first layer, a second layer, a third layer and a fourth layer of the four catalytic layers, wherein the VTiO catalyst of each of the four catalytic layers comprises vanadium oxide (V₂O₅), titanium oxide (TiO₂) , and cesium oxide (Cs₂O) and antimony oxide (Sb₂O₃) as promoters.

However, there is still a need for improved catalysts for the oxidation of o-xylene and/or naphthalene, which make it possible to maximize catalytic performance in terms of yield of phthalic anhydride and which keep this performance as unaltered as possible, i.e. without degrading, over the lifespan of the catalytic bed.

The aim of the present invention is therefore to provide a catalytic system for the oxidation of o-xylene and/or naphthalene to phthalic anhydride that overcomes the drawbacks of conventional catalytic systems.

Within this aim, an object of the invention is to provide a multilayer catalytic bed for producing phthalic anhydride that is characterized by an increased yield of phthalic anhydride and, at the same time, by a reduced decrease in catalytic performance as the lifespan of the catalysts in its layers increases.

Another object of the invention is to provide a catalytic bed for the oxidation of o-xylene and/or naphthalene to phthalic anhydride that can be used in existing reactors without the need for modifying such reactors and/or for making changes to the reaction conditions conventionally used in the industry to produce phthalic anhydride.

Finally, another object of the invention is to provide a process for producing phthalic anhydride with high yield and selectivity.

This aim and these and other objects which will become better apparent hereinafter are achieved by a catalytic bed for the production of phthalic anhydride by oxidation of a hydrocarbon selected from the group consisting of o-xylene, naphthalene and mixtures thereof, said catalytic bed comprising at least four catalytic layers of vanadium and titanium mixed oxide (VTiO) catalyst arranged in series with respect to the flow of a gaseous feed mixture comprising the hydrocarbon and an oxygen-containing gas, so that the gaseous feed mixture flows in sequence through a first layer, a second layer, a third layer and a fourth layer of the four catalytic layers,
wherein the first layer has a length of from 20% to 27% of the total length of the catalytic bed, the second layer has a length of from 37% to 43% of the total length of the catalytic bed, the third layer has a length of from 8% to 14% of the total length of the catalytic bed, and the fourth layer has a length of from 16% to 35% of the total length of the catalytic bed,
wherein the VTiO catalyst of each one of the four catalytic layers comprises vanadium oxide (V₂O₅), titanium oxide (TiO₂), and optionally a first promoter selected from cesium oxide (Cs₂O), antimony oxide (Sb₂O₃), and mixtures thereof, and
wherein the VTiO catalyst of the first layer and the VTiO catalyst of the second layer further comprise a second promoter, the second promoter of the VTiO catalyst of the first layer being copper oxide (CuO) in an amount of from 0.01% to 2% by weight on the total weight of the VTiO catalyst of the first layer, and the second promoter of the VTiO catalyst of the second layer being molybdenum oxide (MoO₃) in an amount of from 0.01% to 2% by weight on the total weight of the VTiO catalyst of the second layer.

The above aim and objects are further achieved by a process for producing phthalic anhydride by oxidation of a hydrocarbon selected from the group consisting of o-xylene, naphthalene and mixtures thereof, comprising the step of feeding a gaseous mixture comprising the hydrocarbon and an oxygen-containing gas into a reactor loaded with a catalytic bed according to the invention in any of the embodiments thereof described herein.

Further characteristics and advantages of the invention will become better apparent from the detailed description that follows and from the accompanying drawings wherein:
Figure 1 is a chart showing the results of the study of the oxidation kinetics of o-xylene conducted in Example 1;
Figure 2 is a chart showing the distribution of the products of the oxidation reaction during the study of the kinetics conducted in Example 1.

As a result of the research conducted by the inventors of the present invention, it was possible to identify a combination of technical characteristics that offers improved catalytic performance over the current generation of catalytic systems for the production of phthalic anhydride and makes it possible to achieve the set aim and objects.

In particular, the present invention relates to a catalytic bed for the production of phthalic anhydride by oxidation of a hydrocarbon selected from the group consisting of o-xylene, naphthalene and mixtures thereof, which has the three characteristics discussed below, by virtue of which the catalytic bed has, with respect to conventional catalytic systems, a greater yield of phthalic anhydride and a reduced decrease in the catalytic performance as the lifespan increases.

On the basis of the first of these characteristics, the catalytic bed of the invention comprises at least four catalytic layers of vanadium and titanium mixed oxide (VTiO) catalyst and such layers are arranged in series with respect to the flow of a gaseous feed mixture comprising o-xylene and/or naphthalene and an oxygen-containing gas (preferably air). The layers are therefore arranged in such a manner that the gaseous feed mixture flows in sequence through a first layer, a second layer, a third layer and a fourth layer of the four catalytic layers.

In other words, the above arrangement in series implies that the gaseous feed mixture, once introduced into the reactor in which the catalytic bed is loaded, flows in sequence (from the inlet of the reactor) through the first catalytic layer, from this first layer to the second catalytic layer, from the second layer to the third catalytic layer, and then from the third layer to the fourth catalytic layer.

As a second characteristic, in the catalytic bed according to the invention the length of the at least four layers of VTiO catalyst has been optimized over the current generation of catalytic beds. Specifically, according to the present invention the first layer has a length of from 20% to 27% of the total length of the catalytic bed, the second layer has a length of from 37% to 43% of the total length of the catalytic bed, the third layer has a length of from 8% to 14% of the total length of the catalytic bed, and the fourth layer has a length of from 16% to 35% of the total length of the catalytic bed.

In a preferred embodiment, the first layer has a length of from 22% to 24% of the total length of the catalytic bed, the second layer has a length of from 40% to 42% of the total length of the catalytic bed, the third layer has a length of from 12% to 14% of the total length of the catalytic bed, and the fourth layer has a length of from 20% to 26% of the total length of the catalytic bed.

In a more preferred embodiment, the first layer has a length equal to 23% of the total length of the catalytic bed, the second layer has a length equal to 41% of the total length of the catalytic bed, the third layer has a length equal to 13% of the total length of the catalytic bed, and the fourth layer has a length equal to 23% of the total length of the catalytic bed.

Although the catalytic bed of the present invention can comprise more than the aforementioned four layers of VTiO catalyst, for example five or more layers, in an embodiment the catalytic bed instead consists of only four catalytic layers, where the first layer constitutes 20-27% of the total length of the catalytic bed, the second layer constitutes 37-43% of the total length of the catalytic bed, the third layer constitutes 8-14% of the total length of the catalytic bed, and the fourth layer constitutes 16-35% of the total length of the catalytic bed. In this embodiment, preferably the first layer constitutes 22-24% of the total length of the catalytic bed, the second layer constitutes 40-42% of the total length of the catalytic bed, the third layer constitutes 12-14% of the total length of the catalytic bed, and the fourth layer constitutes 20-26% of the total length of the catalytic bed, and more preferably the first layer constitutes 23% of the total length of the catalytic bed, the second layer constitutes 41% of the total length of the catalytic bed, the third layer constitutes 13% of the total length of the catalytic bed, and the fourth layer constitutes 23% of the total length of the catalytic bed.

As the third characteristic, in the catalytic bed of the present invention the VTiO catalyst of each one of the four catalytic layers comprises vanadium oxide (V₂O₅), titanium oxide (TiO₂) and optionally a first promoter selected from cesium oxide (Cs₂O), antimony oxide (Sb₂O₃), and mixtures thereof. In order to obtain the desired catalytic performance, the composition of the catalyst of specific layers of the bed has been modified by adding very specific promoters in well-defined quantities. In particular, according to the present invention the VTiO catalyst of the first layer and the VTiO catalyst of the second layer further comprise a second promoter, wherein the second promoter of the VTiO catalyst of the first layer is copper oxide (CuO) in an amount of from 0.01% to 2%, preferably from 0.05% to 1%, more preferably 0.1%, by weight on the total weight of the VTiO catalyst of the first layer, and the second promoter of the VTiO catalyst of the second layer is molybdenum oxide (MoO₃) in an amount of from 0.01% to 2%, preferably from 0.1% to 1%, more preferably 0.5%, by weight on the total weight of the VTiO catalyst of the second layer.

Each one of the VTiO catalysts of the layers of the catalytic bed comprises vanadium oxide in its conventional form of vanadium pentoxide (V₂O₅), preferably in a quantity of from 2% to 15% by weight, more preferably from 5% to 10% by weight, of the total weight of the catalyst. In the preparation of the VTiO catalysts, vanadium oxide can be added as-is or alternatively in the form of a precursor, such as for example ammonium metavanadate, vanadium chloride, vanadium oxychloride, vanadyl acetylacetonate, vanadium alkoxides or other commercially-known and used compounds.

As noted above, each one of the VTiO catalysts of the layers of the catalytic bed of the invention further comprises titanium oxide (TiO₂). This compound is present in preponderant quantities in VTiO catalysts, therefore in a quantity sufficient to reach 100% of the total weight of the catalyst. In the preparation of the catalysts, titanium oxide is preferably used in the crystalline form of anatase, preferably having a surface area of from 10 to 30 m²/g, more preferably from 17 to 25 m²/g.

Each one of the VTiO catalysts of the layers of the catalytic bed can further comprise a first promoter consisting of cesium oxide (Cs₂O) and/or antimony oxide (Sb₂O₃).

If present, cesium oxide is in a maximum quantity of 2% by weight of the total weight of the catalyst, preferably in an amount of from 0.01% to 2%, more preferably from 0.01% to 0.8%, by weight on the total weight of the catalyst. In the preparation of the catalysts, cesium oxide can be added as-is or as its precursor, such as for example cesium sulfate, cesium nitrate, cesium chloride, or other commercial salts or compounds of cesium.

In turn, if present, antimony oxide is in a maximum quantity of 5% by weight of the total weight of the catalyst, preferably in an amount of from 0.5% to 5%, more preferably from 0.5% to 3%, by weight on the total weight of the catalyst. In the preparation of the catalysts, antimony oxide can be added as-is or as its precursor, for example antimony chloride, antimony trisulfide (Sb₂S₃), antimony sulfate, or other commercial salts or compounds of antimony.

Independently of each other, the VTiO catalysts of the layers of the catalytic bed of the invention can also comprise, in addition to the above ingredients, one or more further promoters selected from the group consisting of lithium, potassium, rubidium, aluminum, zirconium, iron, nickel, cobalt, manganese, silver, chromium, tungsten, iridium, tantalum, niobium, arsenic, cerium, and phosphorous. When used, the quantity of these further promoters does not exceed 5% by weight of the total weight of the catalyst.

In a preferred embodiment, the VTiO catalyst of each one of the four catalytic layers comprises, with respect to the total weight of the VTiO catalyst:
- V₂O₅ from 2% to 15% by weight;
- Cs₂O up to 2% by weight;
- Sb₂O₃ up to 5% by weight; and
- TiO₂ as required to reach 100% of the total weight of the VTiO catalyst,
where - in accordance with what is explained above - the VTiO catalyst of the first layer comprises, as second promoter, CuO from 0.01% to 2%, preferably 0.1%, by weight on the total weight of the VTiO catalyst of the first layer, and the VTiO catalyst of the second layer comprises, as second promoter, MoO₃ from 0.01% to 2%, preferably 0.5%, by weight on the total weight of the VTiO catalyst of the second layer.

In this preferred embodiment, the VTiO catalyst of each one of the four catalytic layers can comprise from 0.01% to 2%, preferably from 0.01% to 0.8%, by weight of Cs₂O, and from 0.5% to 5%, preferably from 0.5% to 3%, by weight of Sb₂O₃, each with respect to the total weight of the VTiO catalyst.

In a more preferred embodiment, the VTiO catalyst of each one of the four catalytic layers comprises, with respect to the total weight of the VTiO catalyst:
- V₂O₅ from 5% to 10% by weight;
- Cs₂O from 0.01% to 0.8% by weight;
- Sb₂O₃ from 0.5% to 3% by weight; and
- TiO₂ as required to reach 100% of the total weight of the VTiO catalyst,
wherein the VTiO catalyst of the first layer comprises 0.1% by weight of CuO on the total weight of the VTiO catalyst of the first layer, and the VTiO catalyst of the second layer comprises 0.5% by weight of MoO₃ on the total weight of the VTiO catalyst of the second layer.

For commercial applications, the VTiO catalyst of each layer of the catalytic bed is advantageously supported, i.e. the catalyst is applied, for example as a coating, on an inert support.

Any material that has sufficient chemical inertia and stability can be used as the support. In particular, a support material can be used that is selected from the group consisting of corundum, steatite, alumina, silicon carbide, silica, magnesium oxide, aluminum silicate, and mixtures thereof. Preferably the inert support has the form of particles, in particular granules (spherical or irregular), pellets, cylinders or rings. The VTiO catalyst of each one of the four catalytic layers can be coated on an inert support in an amount of from 2% to 15% by weight, preferably from 3% to 12% by weight, more preferably 8% by weight, with respect to the total weight of the VTiO catalyst and of the support (i.e. the final weight of the supported catalyst).

The VTiO catalyst of each layer of the catalytic bed can be prepared using conventional methods, for example according to the following general process:
1) Preparing a mixture of the active ingredients of the catalyst (vanadium oxide, titanium oxide and, if specified, cesium oxide, antimony oxide, copper oxide, molybdenum oxide), or of precursors thereof that are capable of being converted by heat treatment into the abovementioned ingredients, by dissolving, dispersing or suspending such ingredients or precursors in an aqueous or organic solvent, in which the ingredients or precursors are soluble or dispersible.
2) If a supported catalyst is being prepared, the solution or slurry obtained as above is applied on the inert support, for example in the form of a thin layer of coating, and dried. Coating on the support can be done by spraying the solution or slurry containing the ingredients or their precursors on the support. The operation can be carried out in a heated drum, kept at a suitable temperature for evaporating the solvent, for example in the range of 50-250 °C.

If it is instead desired to prepare an unsupported catalyst, the solvent contained in the solution or slurry can be simply allowed to evaporate and the resultant solid residue is left to dry and/or crumbled, if necessary.

3) The supported or unsupported product as obtained above is then subjected to a thermal treatment for activation, in order to obtain the catalyst. Such thermal treatment can be done in the same heated drum as mentioned above, or in a separate oven, or directly in the reactor used to produce the phthalic anhydride. The thermal treatment is performed according to methods known to the person skilled in the art, in air or in another suitable (non-reducing) atmosphere, at a temperature that varies typically from 150 to 450 °C. By way of example, the thermal treatment can be performed in air and comprises the following steps:
1) heating from ambient temperature to 170°C at a rate of 10°C/min;
2) heating from 170°C to 410°C at a rate of 8°C/min;
3) maintaining the temperature at 410°C for 48h;
4) cooling to 380°C.

In accordance with the present invention, the catalytic bed according to any of its embodiments described above is used for the production of phthalic anhydride by oxidation of a hydrocarbon selected from o-xylene, naphthalene and mixtures of the two, preferably o-xylene.

The oxidation reaction is typically carried out in multitubular fixed-bed reactors. The VTiO catalysts that constitute the layers of the catalytic bed are loaded into the tubes of the reactor and a gaseous mixture of the hydrocarbon (o-xylene and/or naphthalene) with air (or oxygen or another oxygen-containing gas) is passed over the bed.

The initial concentration of hydrocarbon in the gaseous mixture (i.e. the concentration in the reactor feed) is preferably in the range of 1.0-1.8 mol%.

Preferably, the oxidation reaction is carried out at a temperature of from 350 to 450 °C. The pressure at the reactor inlet is preferably from 100 kPa to 200 kPa.

The invention will now be described with reference to the following non-limiting examples.

### EXAMPLE 1 - STUDY OF THE REACTION KINETICS

A study of the oxidation reaction kinetics for the synthesis of phthalic anhydride was carried out, with the aim of investigating the composition of the reaction products at different depths of the catalytic bed and therefore at different contact times.

The study was carried out on a pilot plant, by loading the reaction tube with a current-generation VTiO catalyst, marketed under the name "Polycat PA PO" (Polynt S.p.A.; Scanzorosciate, Italy), so as to obtain a catalytic bed with four layers having the height given in Table 1. The active phase of the catalyst of each layer consists of the same oxides (V₂O₅, TiO₂, Cs₂O and Sb₂O₃), but in different percentages depending on the layer.

**Table 1**

| ***Layer*** | ***Height*** | ***Weight*** |
|---|---|---|
| 1 | 41% (124 cm) | 574 g |
| 2 | 13% (40 cm) | 182 g |
| 3 | 23% (68 cm) | 322 g |
| 4 | 23% (68 cm) | 322 g |
| *Total* | 100% (300 cm) | 1400 g |

The arrangement of the catalytic layers is such that the gaseous feed mixture containing o-xylene as the starting hydrocarbon, once introduced into the catalytic tube, flowed through the layers in the order 1 → 2 → 3 → 4.

In more detail, the study was executed by placing a metallic sheath inside the reaction tube at different depths in the catalytic bed; in this manner at each depth a dedicated catalytic test takes place. For each individual catalytic test, there was a period of ramp-up and optimization of the operating parameters, which was necessary in order to arrive at the standard conditions in which the catalyst is usually employed, and in order to obtain a gaseous flow with a homogeneous and stable composition in output from the catalytic bed. With the optimal conditions reached and the reaction stabilized, a valve arranged on the final part of the sheath was opened so as to allow the gases to be sampled.

The composition of the reaction products taken at different depths of the catalytic bed, and therefore at different contact times, was analyzed with a gas chromatograph. In particular, non-condensable gases were transferred directly to the gas chromatograph and analyzed, while condensable gases were first condensed in acetone at the temperature of -78°C and then analyzed with the gas chromatograph.

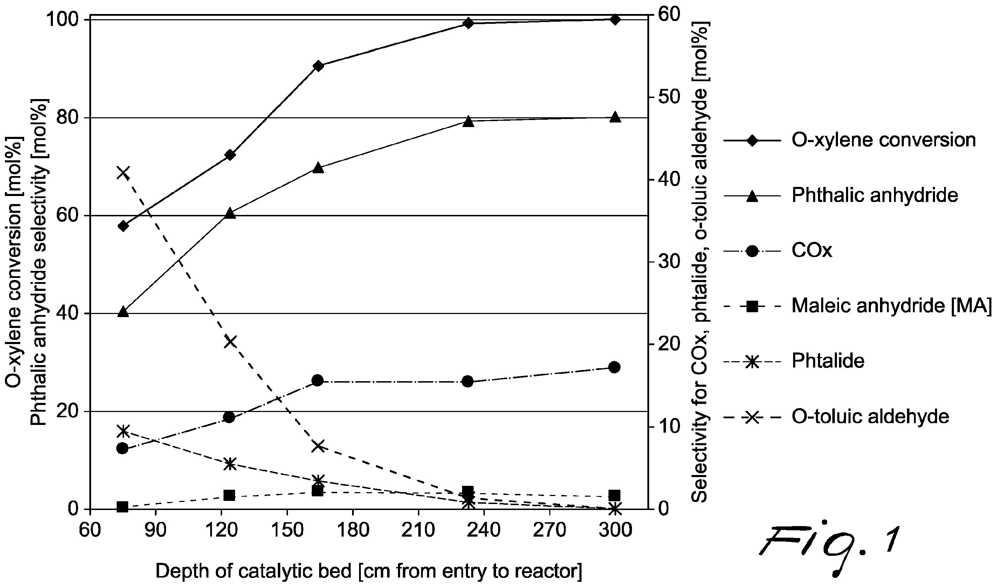

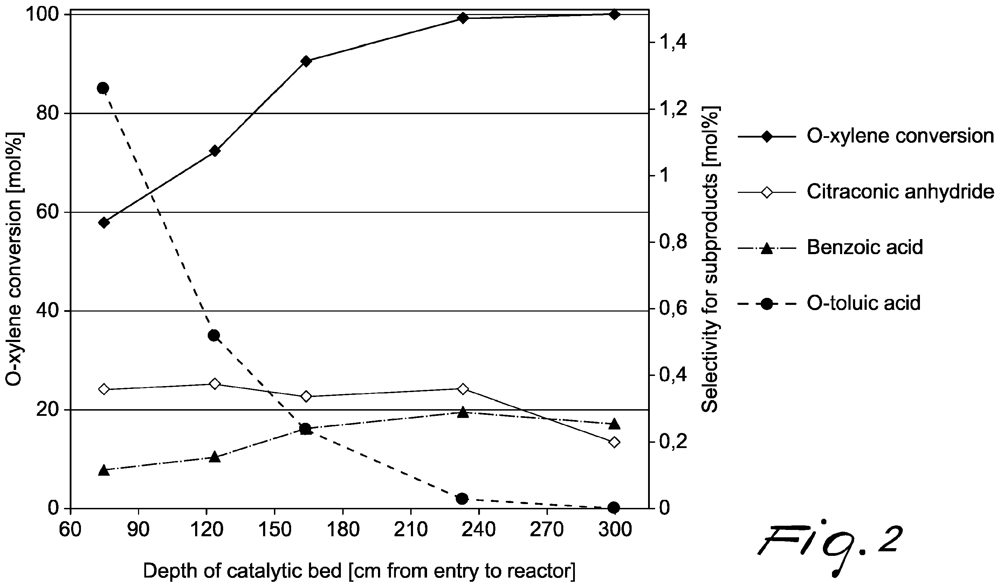

The results, shown in graphic form in Figures 1 and 2, are generally in agreement with the scientific literature. Specifically, as the depth of the catalytic bed increases, the conversion of o-xylene increases progressively until it shows values higher than 99.9 mol% in output from the bed. Even though the final conversion is near-total, it should be noted that in the gaseous flow sampled after having passed through approximately 40% of the catalytic bed, the conversion of the starting hydrocarbon is already higher than 70 mol%.

With regard to the distribution of the reaction products, the primary selective oxidation product is confirmed to be o-toluic aldehyde, the selectivity of which at low contact times is the highest, higher than 40 mol %, and then falls drastically as the rate of conversion increases. The o-toluic acid, obtained by consecutive selective oxidation of o-toluic aldehyde, shows an entirely similar trend, but with lower absolute values. This is ascribable to the high speed of the selective oxidation reaction that converts the o-toluic acid to phthalic anhydride. Finally, phtalide is also considered among the primary products of the reaction, and is obtained in quantities of around 10 mol% at low contact times and is then progressively oxidized to phthalic anhydride as the rate of conversion increases.

According to what was observed for the main intermediates of the selective oxidation reaction, phthalic anhydride instead exhibits a selectivity that increases as the contact time increases. The intermediates are in fact progressively converted until they are transformed into the product of interest.

The two main byproducts of over-oxidation, maleic anhydride and carbon oxides (COₓ), exhibit a low selectivity for low contact times. The selectivity for such byproducts then tends to increase, stabilizing once the first 40% of the catalytic bed has been passed.

Similarly to what has been observed and described previously for the conversion of the starting hydrocarbon, the distribution of the reaction intermediates, of phthalic anhydride and of the products of over-oxidation also shows maximum change after having passed through around the first 40% of the catalytic bed.

Based on the experimental results obtained from studying the kinetics, the inventors of the present invention have concluded that the first layer of the catalytic bed, i.e. the layer at the entry point to the reaction tube with a length equal to approximately 40% of the total, corresponds to the portion of bed through which the flow fed at entry undergoes the greatest change in composition. For this reason, in order to optimize the composition of the catalytic bed, the inventors have studied the effect of adding specific promoters to the composition of the VTiO catalyst of this layer, while keeping all the other parameters unchanged.

### EXAMPLE 2 - PREPARATION OF THE CATALYSTS

A series of VTiO catalysts was prepared having the compositions given in Table 2 in order to study the effect of adding a specific promoter selected from copper oxide (CuO) and molybdenum oxide (MoO₃) to the composition of the current-generation commercial VTiO catalyst. To this end, all the following catalysts were prepared by following the same procedure given below.

The synthesis of each catalyst entails the steps of (i) wet milling, (ii) addition of an organic binder and homogenization of the slurry, and (iii) coating on an inert support.

For the wet milling step, the oxides and the precursors of the metals were loaded in the predefined quantities into a mill. For example, for the preparation of the first layer of the reference catalyst, the following were weighed: 137.37 g of TiO₂, 9.46 g of V₂O₅, 1.25 g of Cs₂SO₄ and 4.20 g of Sb₂O₃. For catalysts that also contain CuO and MoO₃, addition of the promoter was always done during the milling step. After this, 450 g of demineralized water were added into the chamber of the mill, and the milling process was started and kept going for a total time of 16 hours at ambient temperature. At the end of the milling time, the slurry consisting of water and oxides was recovered using additional 200 g of water, necessary to wash the inner walls of the mill, and collected in a beaker.

In the next step, 60 g of VINAVIL^{®} EVA 4612 organic binder (VINAVIL S.p.A.; Milano, Italy) were added to the slurry, and then the final homogenization of the suspension was carried out by magnetic agitation at ambient temperature, for a total time of 30 minutes.

In the third step, the slurry containing the components of the active phase of the catalyst was coated on an inert support consisting of ceramic steatite rings. This operation was carried out by spraying the slurry on the support through a nozzle inside a heated rotating oven, so as to obtain a uniform covering on the entire surface of the rings of the support. During this operation, the slurry composed of water, organic binder and the mixture of oxides was kept at the temperature of 50°C. An amount of 1600 g of ceramic rings (material: steatite - dimensions: outside diameter 7 mm, height 7 mm, thickness 1.5 mm) was loaded into the oven and heated to the temperature of 70°C. Once the temperatures, both of the slurry and of the rings, have homogenized inside the rotating oven, the slurry was fed to the nozzle, coating the ceramic rings with a quantity of active phase equal to 8% by weight on the total weight of the catalytic material consisting of the catalyst and the support. For this operation the nozzle was fed with compressed air at 200 kPa and a flow rate of 750 ml/min.

At the end of the coating step, the temperature of the oven was raised to 100°C, so as to ensure the drying of the rings. The supported catalyst thus obtained is ready to be loaded into the pilot reactor.

By following the synthetic procedure just described, the catalysts listed in Table 2 were prepared.

**Table 2**

| Cat. | Composition of the active phase (wt%) | | | | | | |
|---|---|---|---|---|---|---|---|
| | TiO₂ | SSA TiO₂ [m²/g] | V₂O₅ | Cs₂O | Sb₂O₃ | MoO₃ | CuO |
| 1 | 90.37 | 25 | 6.23 | 0.64 | 2.76 | - | - |
| 2 | 91.36 | 17 | 5.59 | 0.28 | 2.77 | - | - |
| 3 | 90.78 | 17 | 6.16 | 0.14 | 2.92 | - | - |
| 4 | 89.92 | 17 | 9.57 | 0.01 | 0.5 | - | - |
| 5 | 93.13 | 25 | 6.23 | 0.64 | - | - | - |
| 6 | 92.77 | 25 | 6.23 | - | - | - | 1.00 |
| 7 | 93.03 | 25 | 6.23 | 0.64 | - | - | 0.10 |
| 8 | 90.27 | 25 | 6.23 | 0.64 | 2.76 | - | 0.10 |
| 9 | 90.17 | 25 | 6.23 | 0.64 | 2.76 | - | 0.20 |
| 10 | 90.32 | 25 | 6.23 | 0.64 | 2.76 | - | 0.05 |
| 11 | 92.77 | 25 | 6.23 | - | - | 1.00 | - |
| 12 | 93.03 | 25 | 6.23 | 0.64 | - | 0.10 | - |
| 13 | 92.88 | 25 | 6.23 | 0.64 | - | 0.25 | - |
| 14 | 90.12 | 25 | 6.23 | 0.64 | 2.76 | 0.25 | - |
| 15 | 89.87 | 25 | 6.23 | 0.64 | 2.76 | 0.50 | - |

| | | | | | | | |
|---|---|---|---|---|---|---|---|
| SSA = Specific Surface Area | | | | | | | |

### EXAMPLE 3 - CATALYTIC TESTS (1st PART)

A series of tests of the catalytic performance was carried out on a single-tube pilot plant, loading a total quantity of catalytic material equal to 1400 grams and a bed length of 300 cm. The distribution of the four layers of the catalytic bed in terms of length and weight is identical to that shown in Table 1 and corresponds to the configuration of the current generation of catalytic beds for the industrial production of phthalic anhydride from o-xylene.

The catalytic material loaded into the reactor was calcined (activation) *in situ,* to remove the organic binder used in the synthesis and to stabilize the oxides of the active phase. The activation was carried out in air (flow rate 700 Nl/h) with the following steps:
1) heating from ambient temperature to 170°C at a rate of 10°C/min;
2) heating from 170°C to 410°C at a rate of 8°C/min;
3) maintaining temperature at 410°C for 48h;
4) cooling to 380°C.

After the calcination, the reactor was brought to stable running conditions, under which the catalytic performance is evaluated, by following a suitable ramp-up procedure, in which the quantity of o-xylene fed in is progressively increased and the operating parameters are optimized in parallel (flow rate of air and temperature of the bath of molten salts). Table 3 shows the operating conditions under which all the catalytic tests that will be discussed below were carried out.

**Table 3**

| **Operating parameter** | **Unit** | **Typical value** |
|---|---|---|
| Length of catalytic bed | cm | 300 |
| Weight of catalysts | g | 1400 |
| Air flow | Nl/h | 4000 |
| O-xylene feeding | g/h | 320 |
| Concentration of o-xylene | g/Nm³ | 80 |
| Salt bath temperature (SBT) | °C | 350-360 |

Table 4 lists the tests carried out on the pilot plant with the corresponding composition of the loaded catalytic bed (as a combination of the catalysts listed in Table 2) in order to study the effect of the addition of CuO or MoO₃. Table 4 also shows the catalytic performance levels obtained under stable conditions, specifically with reference to the optimal temperature of the bath of salts ("SBT"), the weight yield of phthalic anhydride ("PA Yield"), the unconverted o-xylene in output from the reactor ("OX slippage") and the quantity of phtalide ("PHD") present in the raw phthalic anhydride.

**Table 4**

| **Test** | **Bed configuration** | | | | **Catalytic performance** | | | |
|---|---|---|---|---|---|---|---|---|
| | **Catalytic layers** | | | | **SBT (°C)** | **PA Yield (wt%)** | **OX slippage (mg/Nm³)** | **PHD (wt%)** |
| | **1** | **2** | **3** | **4** | | | | |
| A | Cat1 | Cat2 | Cat3 | Cat4 | 353 | 114.6 | 20 | 0.02 |
| B | Cat5 | Cat2 | Cat3 | Cat4 | 380 | 111.3 | 5 | 0.01 |
| C | Cat6 | Cat2 | Cat3 | Cat4 | 342 | 102.7 | 1 | 0.01 |
| D | Cat7 | Cat2 | Cat3 | Cat4 | 356 | 112.4 | 60 | 0.01 |
| E | Cat8 | Cat2 | Cat3 | Cat4 | 350 | 115.1 | 400 | 0.2 |
| F | Cat9 | Cat2 | Cat3 | Cat4 | 350 | 114.5 | 50 | 0.02 |
| G | Cat10 | Cat2 | Cat3 | Cat4 | 357 | 113.7 | 10 | 0.01 |
| H | Cat11 | Cat2 | Cat3 | Cat4 | 347 | 104.3 | 1 | 0.01 |
| I | Cat12 | Cat2 | Cat3 | Cat4 | 380 | 110.5 | 10 | 0.01 |
| L | Cat13 | Cat2 | Cat3 | Cat4 | 360 | 112.7 | 10 | 0.01 |
| M | Cat14 | Cat2 | Cat3 | Cat4 | 348 | 114.7 | 60 | 0.03 |
| N | Cat15 | Cat2 | Cat3 | Cat4 | 350 | 115.8 | 30 | 0.01 |

*Reference test*: the catalytic test A is used as a reference for comparison with all the other tests, since this test was carried out using the current industrial generation of catalytic bed for the production of phthalic anhydride. Specifically, in the pilot reactor used in this example the reference bed showed a yield of phthalic anhydride equal to 114.6 wt% and values of unconverted o-xylene and phtalide content in the raw phthalic anhydride respectively of 20 mg/Nm³ and 0.02 wt%. During running, the hot spot of the reaction was identified in the first layer, a position deemed optimal.

*CuO promoter*: the catalytic tests from C to G, together with tests A and B, have made it possible to evaluate the effect of the addition of CuO as a promoter of the VTiO catalyst of the first layer of the bed.

First of all, the CuO promoter increased the catalytic activity of the VTiO phase (see tests B, C and D), both in the absence of both promoters of the standard configuration (cesium and antimony), and in the presence of cesium, which is notoriously an element that inhibits catalytic activity because it promotes selectivity. Specifically, from tests B, C, D it was seen that the addition of a certain quantity of CuO made it possible to stabilize the hot spot of the reaction in the first layer of the bed, thereby contrasting the inhibiting effect of cesium.

Furthermore, the effect of the same quantity of CuO added in the presence of both the cesium and antimony promoters has made it possible to increase the selectivity of the entire catalytic bed. In fact, test E, loaded with catalyst 8 as the first layer of the bed, obtained a greater yield of phthalic anhydride than the reference test (115.1 against 114.6), notwithstanding an experimental condition of lower conversion as indicated by the greater quantity of unconverted o-xylene monitored in output from the reactor.

A further indication of the greater selectivity of the entire catalytic bed of test E is shown by the distribution analysis of the reaction products in output from the reactor, the values of which are shown in Table 5. From comparison with the reference test A, it should be noted that in test E a greater quantity of unconverted o-xylene was detected, together with greater quantities of phtalide and o-toluic aldehyde (i.e. the two main intermediates of the selective oxidation path).

**Table 5**

| Test | Products in output from the reactor (wt%) | | | | | | | | |
|---|---|---|---|---|---|---|---|---|---|
| | AcA | MA | CA | BAc | PA | PHD | OX | oTAc | oTA |
| A | 0.29 | 3.35 | 0.29 | 0.65 | 95.31 | 0.06 | 0.02 | 0.01 | 0.02 |
| E | 0.35 | 3.25 | 0.31 | 0.49 | 95.17 | 0.22 | 0.11 | 0.01 | 0.09 |
| N | 0.38 | 3.41 | 0.26 | 0.56 | 95.28 | 0.05 | 0.02 | 0.01 | 0.03 |

| | | | | | | | | | |
|---|---|---|---|---|---|---|---|---|---|
| (AcA = acetic acid; MA = maleic anhydride; CA = citraconic anhydride; BAc = benzoic acid; PA = phthalic anhydride; PHD = phtalide; OX = o-xylene; oTAc = o-toluic acid; oTA = o-toluic aldehyde). | | | | | | | | | |

*MoO₃* promoter: the catalytic tests from H to N, together with tests A and B, have made it possible to evaluate the effect of the addition of MoO₃ as a promoter of the VTiO catalyst of the first layer of the bed.

Also MoO₃ increased the catalytic activity of the VTiO phase (see tests H, I and L), in the absence of both promoters of the standard configuration (cesium and antimony), as well as in the presence of cesium, which as mentioned above is an inhibitor of activity because it promotes selectivity. Specifically, from tests H, I, L it was seen that the addition of a certain quantity of MoO₃ made it possible to stabilize the hot spot of the reaction in the first layer of the bed, thereby contrasting the inhibiting effect of cesium.

Furthermore, the addition of MoO₃ also increased selectivity, as revealed by tests M and N. In fact, in test M the addition of molybdenum in the first layer resulted in a yield of phthalic anhydride greater than that of the reference test A (115.8 against 114.6), without any substantial change in the unconverted o-xylene, in the quantity of phtalide in the raw product, and in the distribution of the reaction products (Table 5).

The inventors of the invention have therefore concluded that:
- the addition of CuO in the first layer results in obtaining a catalytic bed with a greater selectivity than the standard, by virtue of the (albeit limited) increase in the yield of phthalic anhydride associated with the greater quantity of reaction intermediates generated and with the lower conversion; and
- the addition of MoO₃ acts both as a promoter of activity and of selectivity of the catalytic bed.

### EXAMPLE 4 - CATALYTIC TESTS (2ND PART)

In light of the above results, the inventors of the invention have decided to exploit the characteristic of the layer containing CuO to generate a high quantity of reaction intermediates that could then be selectively converted to phthalic anhydride through a layer containing MoO₃ and to limit the overall formation of over-oxidation products such as COₓ and maleic anhydride, so as to increase the catalytic performance of the process by virtue of a possible synergistic effect of the use of both promoters, CuO and MoO₃, in the catalytic bed.

Further tests were therefore conducted in the pilot plant in which both the length of the layers of the catalytic bed and the composition of the catalysts were changed.

Table 6 shows the configuration of the layers (length and quantity of catalyst loaded) used for these further catalytic tests, comparing it with the configuration of the reference standard used in the previous tests A-N.

**Table 6**

| Layer | Standard configuration (tests A÷N) | | Invention configuration (tests O÷Q) | |
|---|---|---|---|---|
| | Length (cm) | Weight (g) | Length (cm) | Weight (g) |
| 1 | 124 (41%) | 574 | 68 (23%) | 322 |
| 2 | 40 (13%) | 182 | 124 (41%) | 574 |
| 3 | 68 (23%) | 322 | 40 (13%) | 182 |
| 4 | 68 (23%) | 322 | 68 (23%) | 322 |
| *Total* | 300 | 1400 | 300 | 1400 |

Table 7 on the other hand shows the configuration, in terms of composition of the catalysts, of the layers that were loaded into the pilot plant and the corresponding results obtained.

**Table 7**

| **Test** | **Bed configuration** | | | | **Catalytic performance** | | | |
|---|---|---|---|---|---|---|---|---|
| | **Catalytic layers** | | | | **SBT (°C)** | **PA Yield (wt%)** | **OX slippage (mg/Nm³)** | **PHD (wt%)** |
| | **1** | **2** | **3** | **4** | | | | |
| A | Cat1 | Cat2 | Cat3 | Cat4 | 353 | 114.6 | 20 | 0.02 |
| O | Cat8 | Cat1 | Cat2 | Cat3 | 360 | 115.7 | 500 | 0.13 |
| P | Cat8 | Cat15 | Cat2 | Cat3 | 356 | 116.2 | 250 | 0.15 |
| Q | Cat1 | Cat1 | Cat2 | Cat3 | 373 | 113.8 | 57 | 0.06 |

Test Q was carried out first, in order to evaluate the sole effect of different partitioning of the length of the layers on the catalytic performance. In this test, the first and the second layer of the bed consist of catalyst 1 of Example 2 (i.e. the catalyst prepared according to the standard recipe and containing only cesium and antimony as promoters). Comparison of the results obtained with the reference test A shows a worsening of the catalytic performance in test Q, showing that, in the presence of only the standard promoters cesium and antimony, the new configuration of the layers of the catalytic bed does not bring an improvement of the yield of phthalic anhydride.

Subsequently, in tests O and P the simultaneous use of the CuO and/or MoO₃ promoters was evaluated, together with the new partitioning of the layers. The results of these two tests showed that acting simultaneously on the proportions of the layers of the bed and on the composition of the catalysts obtains a greater yield of phthalic anhydride than the reference test A.

In test O, the addition of CuO to the catalyst of the first layer of the bed and the simultaneous re-proportioning of the length of the layers has in fact shown a yield of phthalic anhydride that exceeds the yield of the reference test A (115.7 wt% against 114.6 wt%). However, in parallel a distribution of byproducts has also been observed that is highly skewed toward the main intermediates of the mechanism (phtalide and o-toluic aldehyde), which is not compatible with the industrially-required quality requirements of the product (Table 8).

**Table 8**

| Test | Products in output from the reactor (wt%) | | | | | | | | |
|---|---|---|---|---|---|---|---|---|---|
| | AcA | MA | CA | BAc | PA | PHD | OX | oTAc | oTA |
| A | 0.29 | 3.35 | 0.29 | 0.65 | 95.31 | 0.06 | 0.02 | 0.01 | 0.02 |
| O | 0.31 | 3.89 | 0.40 | 0.30 | 93.79 | 0.54 | 0.44 | 0.04 | 0.29 |
| P | 0.27 | 3.18 | 0.36 | 0.59 | 95.00 | 0.28 | 0.14 | 0.02 | 0.16 |

| | | | | | | | | | |
|---|---|---|---|---|---|---|---|---|---|
| (AcA = acetic acid; MA = maleic anhydride; CA = citraconic anhydride; BAc = benzoic acid; PA = phthalic anhydride; PHD = phtalide; OX = o-xylene; oTAc = o-toluic acid; oTA = o-toluic aldehyde). | | | | | | | | | |

On the other hand, in test P the addition of CuO to the catalyst of the first layer and the addition of MoO₃ to the catalyst of the second layer with the simultaneous re-proportioning of the length of the layers has shown the most marked improvement in terms of catalytic performance. In fact a yield of phthalic anhydride equal to 116.2 wt% has been found, in contrast to 114.6 wt% for the reference test A. In addition, the distribution of the reaction products, although richer in reaction intermediates than the reference test A, has remained entirely acceptable in terms of the quality requirements of the product on the industrial level.

These tests have therefore shown the presence of a synergistic effect deriving from the addition of CuO and MoO₃ as promoters of the VTiO active phase, simultaneously with a re-proportioning of the catalytic layers (test P), by virtue of which it has been possible to maximize the effect and the benefits of the individual promoters.

### EXAMPLE 5 - LIFESPAN TEST

In light of the superior catalytic performance shown by the catalytic bed of test P, the inventors have extended this test in order to evaluate the deactivation behavior of the catalysts of the various layers as the running time increases, evaluating in particular whether the performance observed at the end of the ramp-up and optimization of the reaction conditions were maintained over time and whether the new configuration showed a deactivation trend comparable to that of the bed of the reference test A.

Table 9 compares the deactivation profile observed for the reference bed of test A and for the bed of test P according to the present invention. Specifically, deactivation is expressed as the average of the yield of phthalic anhydride at regular intervals of 50 daily runs in the pilot plant. The bed of the present invention, in addition to the better performance in terms of yield of phthalic anhydride observed above, also shows a radical improvement in deactivation, in that Table 9 makes it clear that the loss of yield of phthalic anhydride over time, owing to the decrease in activity and selectivity of the catalysts, is much less marked than with the reference bed of test A.

**Table 9**

| *Runs* | Average yield of PA per 50 runs (wt%) | |
|---|---|---|
| | A | P |
| 0-50 | 113.6 | 112.0 |
| 50-100 | 114.5 | 115.6 |
| 100-150 | 114.4 | 115.5 |
| 150-200 | 113.6 | 115.3 |
| 200-250 | 113.3 | 115.1 |
| 250-300 | 112.8 | 114.9 |
| 300-350 | 112.2 | 115.0 |
| 350-400 | 111.7 | 114.5 |
| 400-450 | 111.5 | 114.1 |
| 450-500 | 111.1 | 114.0 |

In practice it has been found that the catalytic bed according to the invention fully achieves the set aim, in that it makes available a catalytic system for the oxidation of o-xylene and/or naphthalene to phthalic anhydride that - with respect to the present generation of catalytic systems - is characterized by an improvement in catalytic performance in terms of increase in the yield of the product of interest (phthalic anhydride) and simultaneously by a reduced decrease in the catalytic performance as the lifespan increases.

Furthermore, it has also been observed that the catalytic bed according to the invention achieves the set aim in that it can be used in existing reactors without the need for changes to those reactors and/or to the reaction conditions conventionally used in industry to produce phthalic anhydride.

Finally, it has also been observed that the catalytic bed of the invention also fulfills the object of making available a process for producing phthalic anhydride that offers high yield and selectivity.

## Claims

1. A catalytic bed for the production of phthalic anhydride by oxidation of a hydrocarbon selected from the group consisting of o-xylene, naphthalene and mixtures thereof, said catalytic bed comprising at least four catalytic layers of vanadium and titanium mixed oxide (VTiO) catalyst arranged in series with respect to the flow of a gaseous feed mixture comprising the hydrocarbon and an oxygen-containing gas, so that the gaseous feed mixture flows in sequence through a first layer, a second layer, a third layer and a fourth layer of the four catalytic layers,
wherein the first layer has a length of from 20% to 27% of the total length of the catalytic bed, the second layer has a length of from 37% to 43% of the total length of the catalytic bed, the third layer has a length of from 8% to 14% of the total length of the catalytic bed, and the fourth layer has a length of from 16% to 35% of the total length of the catalytic bed,
wherein the VTiO catalyst of each one of the four catalytic layers comprises vanadium oxide (V₂O₅), titanium oxide (TiO₂), and optionally a first promoter selected from cesium oxide (Cs₂O), antimony oxide (Sb₂O₃), and mixtures thereof, and
wherein the VTiO catalyst of the first layer and the VTiO catalyst of the second layer further comprise a second promoter, the second promoter of the VTiO catalyst of the first layer being copper oxide (CuO) in an amount of from 0.01% to 2% by weight on the total weight of the VTiO catalyst of the first layer, and the second promoter of the VTiO catalyst of the second layer being molybdenum oxide (MoO₃) in an amount of from 0.01% to 2% by weight on the total weight of the VTiO catalyst of the second layer.

2. The catalytic bed according to claim 1, wherein the first layer has a length of from 22% to 24% of the total length of the catalytic bed, the second layer has a length of from 40% to 42% of the total length of the catalytic bed, the third layer has a length of from 12% to 14% of the total length of the catalytic bed, and the fourth layer has a length of from 20% to 26% of the total length of the catalytic bed, preferably wherein the first layer has a length equal to 23% of the total length of the catalytic bed, the second layer has a length equal to 41% of the total length of the catalytic bed, the third layer has a length equal to 13% of the total length of the catalytic bed, and the fourth layer has a length equal to 23% of the total length of the catalytic bed.

3. The catalytic bed according to claim 1 or 2, consisting of four catalytic layers of VTiO catalyst.

4. The catalytic bed according to any of the preceding claims, wherein the VTiO catalyst of the first layer comprises 0.1% by weight of CuO on the total weight of the VTiO catalyst of the first layer, and the VTiO catalyst of the second layer comprises 0.5% by weight of MoO₃ on the total weight of the VTiO catalyst of the second layer.

5. The catalytic bed according to any of the preceding claims, wherein the VTiO catalyst of each one of the four catalytic layers comprises, with respect to the total weight of the VTiO catalyst:
• V₂O₅ from 2% to 15% by weight;
• Cs₂O up to 2% by weight;
• Sb₂O₃ up to 5% by weight; and
• TiO₂ as required to reach 100% of the total weight of the VTiO catalyst.

6. The catalytic bed according to claim 5, wherein the VTiO catalyst of each one of the four catalytic layers comprises from 0.01% to 2%, preferably from 0.01% to 0.8%, by weight of Cs₂O, and from 0.5% to 5%, preferably from 0.5% to 3%, by weight of Sb₂O₃, each one with respect to the total weight of the VTiO catalyst.

7. The catalytic bed according to any of the preceding claims, wherein the VTiO catalyst of each one of the four catalytic layers comprises, with respect to the total weight of the VTiO catalyst:
• V₂O₅ from 5% to 10% by weight;
• Cs₂O from 0.01% to 0.8% by weight;
• Sb₂O₃ from 0.5% to 3% by weight; and
• TiO₂ as required to reach 100% of the total weight of the VTiO catalyst,
and wherein the VTiO catalyst of the first layer comprises 0.1% by weight of CuO on the total weight of the VTiO catalyst of the first layer, and the VTiO catalyst of the second layer comprises 0.5% by weight of MoO₃ on the total weight of the VTiO catalyst of the second layer.

8. The catalytic bed according to any of the preceding claims, wherein the VTiO catalyst of each one of the four catalytic layers is coated on an inert support, preferably in an amount of from 2% to 15% by weight, more preferably from 3% to 12% by weight, on the total weight of the VTiO catalyst and of the support.

9. The catalytic bed according to any of the preceding claims, wherein the inert support consists of granules (spherical or irregular), pellets, cylinders or rings made of a material selected from the group consisting of corundum, steatite, alumina, silicon carbide, silica, magnesium oxide, aluminum silicate, and mixtures thereof.

10. A process for the production of phthalic anhydride by oxidation of a hydrocarbon selected from the group consisting of o-xylene, naphthalene and mixtures thereof, comprising the step of feeding a gaseous mixture comprising the hydrocarbon and an oxygen-containing gas into a reactor loaded with a catalytic bed according to any of claims 1-9.

## Patentansprüche

1. Ein Katalysatorbett zur Herstellung von Phthalsäureanhydrid durch Oxidation eines Kohlenwasserstoffs, gewählt aus der Gruppe bestehend aus o-Xylen, Naphthalen und Mischungen davon, wobei das Katalysatorbett mindestens vier katalytische Schichten von Vanadium- und Titan-Mischoxid-(VTiO-)Katalysator umfasst, angeordnet in Reihen mit Bezug auf den Strom einer gasförmigen Zuführmischung, die den Kohlenwasserstoff und ein sauerstoffhaltiges Gas umfasst, so dass die gasförmige Zuführmischung nacheinander durch eine erste Schicht, eine zweite Schicht, eine dritte Schicht und eine vierte Schicht der vier katalytischen Schichten strömt,
wobei die erste Schicht eine Länge von 20% bis 27% der Gesamtlänge des Katalysatorbetts hat, die zweite Schicht eine Länge von 37% bis 43% der Gesamtlänge des Katalysatorbetts hat, die dritte Schicht eine Länge von 8% bis 14% der Gesamtlänge des Katalysatorbetts hat und die vierte Schicht eine Länge von 16% bis 35% der Gesamtlänge des Katalysatorbetts hat,
wobei der VTiO-Katalysator jeder der vier katalytischen Schichten Vanadiumoxid (V₂O₅), Titanoxid (TiO₂) und wahlweise einen ersten Promotor umfasst, gewählt aus Cäsiumoxid (Cs₂O), Antimonoxid (Sb₂O₃) und Mischungen davon, und
wobei der VTiO-Katalysator der ersten Schicht und der VTiO-Katalysator der zweiten Schicht weiter einen zweiten Promotor umfassen, wobei der zweite Promotor des VTiO-Katalysators der ersten Schicht Kupferoxid (CuO) in einer Menge von 0,01 Gewichtsprozent bis 2 Gewichtsprozent des Gesamtgewichts des VTiO-Katalysators der ersten Schicht ist und der zweite Promotor des VTiO-Katalysators der zweiten Schicht Molybdänoxid (MoO₃) in einer Menge von 0,01 Gewichtsprozent bis 2 Gewichtsprozent des Gesamtgewichts des VTiO-Katalysators der zweiten Schicht ist.

2. Das Katalysatorbett gemäß Anspruch 1, worin die erste Schicht eine Länge von 22% bis 24% der Gesamtlänge des Katalysatorbetts hat, die zweite Schicht eine Länge von 40% bis 42% der Gesamtlänge des Katalysatorbetts hat, die dritte Schicht eine Länge von 12% bis 14% der Gesamtlänge des Katalysatorbetts hat und die vierte Schicht eine Länge von 20% bis 26% der Gesamtlänge des Katalysatorbetts hat, wobei die erste Schicht vorzugsweise eine Länge gleich 23% der Gesamtlänge des Katalysatorbetts hat, die zweite Schicht eine Länge gleich 41% der Gesamtlänge des Katalysatorbetts hat, die dritte Schicht eine Länge gleich 13% der Gesamtlänge des Katalysatorbetts hat und die vierte Schicht eine Länge gleich 23% der Gesamtlänge des Katalysatorbetts hat.

3. Das Katalysatorbett gemäß Anspruch 1 oder 2, das aus vier katalytischen Schichten des VTiO-Katalysators besteht.

4. Das Katalysatorbett gemäß einem beliebigen der obigen Ansprüche, worin der VTiO-Katalysator der ersten Schicht 0,1 Gewichtsprozent CuO, bezogen auf das Gesamtgewicht des VTiO-Katalysators der ersten Schicht, umfasst und der VTiO-Katalysator der zweiten Schicht 0,5 Gewichtsprozent MoO₃ des Gesamtgewichts des VTiO-Katalysators der zweiten Schicht umfasst.

5. Das Katalysatorbett gemäß einem beliebigen der obigen Ansprüche, worin der VTiO-Katalysator jeder der vier katalytischen Schichten, bezogen auf das Gesamtgewicht des VTiO-Katalysators, Folgendes umfasst:
- V₂O₅ von 2 bis 15 Gewichtsprozent,
- Cs₂O bis zu 2 Gewichtsprozent,
- Sb₂O₃ bis zu 5 Gewichtsprozent und
- TiO₂ je nach Bedarf zum Erreichen von 100% des Gesamtgewichts des VTiO-Katalysators.

6. Das Katalysatorbett gemäß Anspruch 5, worin der VTiO-Katalysator jeder der vier katalytischen Schichten von 0,01 bis 2, vorzugsweise von 0,01 bis 0,8, Gewichtsprozent Cs₂O und von 0,5 bis 5, vorzugsweise von 0,5 bis 3, Gewichtsprozent Sb₂O₃, jeweils bezogen auf das Gesamtgewicht des VTiO-Katalysators, umfasst.

7. Das Katalysatorbett gemäß einem beliebigen der obigen Ansprüche, worin der VTiO-Katalysator jeder der vier katalytischen Schichten, bezogen auf das Gesamtgewicht des VTiO-Katalysators, Folgendes umfasst:
- V₂O₅ von 5 bis 10 Gewichtsprozent,
- Cs₂O von 0,01 bis 0,8 Gewichtsprozent,
- Sb₂O₃ von 0,5 bis 3 Gewichtsprozent und
- TiO₂ je nach Bedarf zum Erreichen von 100% des Gesamtgewichts des VTiO-Katalysators;
und worin der VTiO-Katalysator der ersten Schicht 0,1 Gewichtsprozent CuO des Gesamtgewichts des VTiO-Katalysators der ersten Schicht umfasst und der VTiO-Katalysator der zweiten Schicht 0,5 Gewichtsprozent MoO₃, bezogen auf das Gesamtgewicht des VTiO-Katalysators der zweiten Schicht, umfasst.

8. Das Katalysatorbett gemäß einem beliebigen der obigen Ansprüche, worin der VTiO-Katalysator jeder der vier katalytischen Schichten auf einen inerten Träger, vorzugsweise in einer Menge von 2 bis 15 Gewichtsprozent, stärker bevorzugt 3 bis 12 Gewichtsprozent, des Gesamtgewichts des VTiO-Katalysators und des Trägers aufgetragen ist.

9. Das Katalysatorbett gemäß einem beliebigen der obigen Ansprüche, worin der inerte Träger aus (kugelförmigen oder unregelmäßigen) Körnchen, Pellets, Zylindern oder Ringen aus einem Material besteht, das gewählt ist aus der Gruppe bestehend aus Korund, Steatit, Aluminiumoxid, Siliziumcarbid, Siliziumdioxid, Magnesiumoxid, Aluminiumsilikat und Mischungen davon.

10. Ein Verfahren zur Herstellung von Phthalsäureanhydrid durch Oxidation eines Kohlenwasserstoffs, gewählt aus der Gruppe bestehend aus o-Xylen, Naphthalen und Mischungen davon, das den Schritt des Zuführens einer gasförmigen Mischung, die den Kohlenwasserstoff und ein sauerstoffhaltiges Gas umfasst, in einen Reaktor umfasst, welcher mit einem Katalysatorbett gemäß einem beliebigen der Ansprüche 1-9 geladen ist.

## Revendications

1. Lit catalytique pour la production d'anhydride phtalique par oxydation d'un hydrocarbure choisi dans le groupe constitué par l'o-xylène, le naphtalène et leurs mélanges, ledit lit catalytique comprenant au moins quatre couches catalytiques d'un catalyseur à base d'oxyde mixte de vanadium et de titane (VTiO) disposées en série par rapport à l'écoulement d'un mélange gazeux d'alimentation comprenant l'hydrocarbure et un gaz contenant de l'oxygène, de telle sorte que le mélange gazeux d'alimentation s'écoule successivement à travers une première couche, une deuxième couche, une troisième couche et une quatrième couche des quatre couches catalytiques,
dans lequel la première couche a une longueur comprise entre 20 % et 27 % de la longueur totale du lit catalytique, la deuxième couche a une longueur comprise entre 37 % et 43 % de la longueur totale du lit catalytique, la troisième couche a une longueur comprise entre 8 % et 14 % de la longueur totale du lit catalytique, et la quatrième couche a une longueur comprise entre 16 % et 35 % de la longueur totale du lit catalytique,
où le catalyseur VTiO de chacune des quatre couches catalytiques comprend de l'oxyde de vanadium (V₂O₅), de l'oxyde de titane (TiO₂) et, éventuellement, un premier promoteur choisi parmi l'oxyde de césium (Cs₂O), l'oxyde d'antimoine (Sb₂O₃) et des mélanges de ceux-ci, et
où le catalyseur VTiO de la première couche et le catalyseur VTiO de la deuxième couche comprennent en outre un deuxième promoteur, le deuxième promoteur du catalyseur VTiO de la première couche étant de l'oxyde de cuivre (CuO) en une quantité comprise entre 0,01 % et 2 % en poids par rapport au poids total du catalyseur VTiO de la première couche, et le deuxième promoteur du catalyseur VTiO de la deuxième couche étant de l'oxyde de molybdène (MoO₃) en une quantité comprise entre 0,01 % et 2 % en poids par rapport au poids total du catalyseur VTiO de la deuxième couche.

2. Lit catalytique selon la revendication 1, dans lequel la première couche a une longueur comprise entre 22 % et 24 % de la longueur totale du lit catalytique, la deuxième couche a une longueur comprise entre 40 % et 42 % de la longueur totale du lit catalytique, la troisième couche a une longueur comprise entre 12 % et 14 % de la longueur totale du lit catalytique, et la quatrième couche a une longueur comprise entre 20 % et 26 % de la longueur totale du lit catalytique, de préférence dans lequel la première couche a une longueur égale à 23 % de la longueur totale du lit catalytique, la deuxième couche a une longueur égale à 41 % de la longueur totale du lit catalytique, la troisième couche a une longueur égale à 13 % de la longueur totale du lit catalytique, et la quatrième couche a une longueur égale à 23 % de la longueur totale du lit catalytique.

3. Lit catalytique selon la revendication 1 ou 2, constitué de quatre couches catalytiques de catalyseur VTiO.

4. Lit catalytique selon l'une quelconque des revendications précédentes, dans lequel le catalyseur VTiO de la première couche comprend 0,1 % en poids de CuO par rapport au poids total du catalyseur VTiO de la première couche, et le catalyseur VTiO de la deuxième couche comprend 0,5 % en poids de MoO₃ par rapport au poids total du catalyseur VTiO de la deuxième couche.

5. Lit catalytique selon l'une quelconque des revendications précédentes, dans lequel le catalyseur VTiO de chacune des quatre couches catalytiques comprend, par rapport au poids total du catalyseur VTiO:
• V₂O₅ de 2 % à 15 % en poids;
• Cs₂O jusqu'à 2 % en poids;
• Sb₂O₃ jusqu'à 5 % en poids; et
• TiO₂ en quantité nécessaire pour atteindre 100 % du poids total du catalyseur VTiO.

6. Lit catalytique selon la revendication 5, dans lequel le catalyseur VTiO de chacune des quatre couches catalytiques comprend de 0,01 % à 2 %, de préférence de 0,01 % à 0,8 %, en poids de Cs₂O, et de 0,5 % à 5 %, de préférence de 0,5 % à 3 %, en poids de Sb₂O₃, chacun par rapport au poids total du catalyseur VTiO.

7. Lit catalytique selon l'une quelconque des revendications précédentes, dans lequel le catalyseur VTiO de chacune des quatre couches catalytiques comprend, par rapport au poids total du catalyseur VTiO :
• V₂O₅ de 5 % à 10 % en poids;
• Cs₂O de 0,01 % à 0,8 % en poids;
• Sb₂O₃ de 0,5 % à 3 % en poids; et
• du TiO₂ en quantité nécessaire pour atteindre 100 % du poids total du catalyseur VTiO,
et dans lequel le catalyseur VTiO de la première couche comprend 0,1 % en poids de CuO par rapport au poids total du catalyseur VTiO de la première couche, et le catalyseur VTiO de la deuxième couche comprend 0,5 % en poids de MoO₃ par rapport au poids total du catalyseur VTiO de la deuxième couche.

8. Lit catalytique selon l'une quelconque des revendications précédentes, dans lequel le catalyseur VTiO de chacune des quatre couches catalytiques est revêtu sur un support inerte, de préférence en une quantité comprise entre 2 % et 15 % en poids, plus de préférence entre 3 % et 12 % en poids, par rapport au poids total du catalyseur VTiO et du support.

9. Lit catalytique selon l'une quelconque des revendications précédentes, dans lequel le support inerte est constitué de granules (sphériques ou irréguliers), de pastilles, de cylindres ou d'anneaux en un matériau choisi dans le groupe constitué par le corindon, la stéatite, l'alumine, le carbure de silicium, la silice, l'oxyde de magnésium, le silicate d'aluminium et leurs mélanges.

10. Procédé de production d'anhydride phtalique par oxydation d'un hydrocarbure choisi dans le groupe constitué par l'o-xylène, le naphtalène et leurs mélanges, comprenant l'étape consistant à introduire un mélange gazeux comprenant l'hydrocarbure et un gaz contenant de l'oxygène dans un réacteur chargé d'un lit catalytique selon l'une quelconque des revendications 1 à 9.
